# EUROPÄISCHE PATENTANMELDUNG

(11) **EP 1 444 968 A2**
(43) Veröffentlichungstag der Anmeldung: **11.08.2004**
(21) Anmeldenummer: 04002259.2
(22) Anmeldetag: 02.02.2004
(51) Int. Cl.: A61F 5/451

(54) **Vorrichtung zum Auffangen von Körperflüssigkeiten**

(30) Priorität: 10.02.2003 DE 20302145 U
(71) Anmelder: Jovanov, Mile, 87463 Dietmannsried (DE)
(72) Erfinder: Jovanov, Mile, 87463 Dietmannsried (DE)
(74) Vertreter: Hutzelmann, Gerhard

(57) **Zusammenfassung**

Vorrichtung zum Auffangen von Körperflüssigkeiten, insbesondere von Urin, insbesondere bei unter Inkontinenz leidenden Personen, wobei eine unter der Kleidung zu tragende Einrichtung(2) zum Auffangen des austretenden Urins vorgesehen ist.

## Beschreibung

Die Erfindung bezieht sich auf eine Vorrichtung zum Auffangen von Körperflüssigkeiten, insbesondere von Urin, insbesondere bei unter Inkontinenz leidenden Personen.

Es sind eine Vielzahl derartiger Vorrichtungen bekannt, die jedoch den Nachteil aufweisen, meist nicht wirkungsvoll vor Urinflecken auf Kleidungsstücken zu schützen.

Aufgabe der Erfindung ist es eine Vorrichtung der genannten Art vorzuschlagen, welche die ungewollte Absonderung von Urin an die Umwelt verhindert und dabei die tragende Person in ihrer Bewegungsfreiheit nicht einschränkt.

Diese Aufgabe wird erfindungsgemäß dadurch gelöst, daß eine unter der Kleidung zu tragende Einrichtung zum Auffangen des austretenden Urins vorgesehen ist.

Dabei hat es sich als sehr vorteilhaft erwiesen, wenn ein Sammelbehälter zum Speichern des aufgefangenen Urins vorgesehen ist.

Die Auffangeinrichtung nimmt den austretenden Urin auf und leitet ihn an den Sammelbehälter weiter, wo er gespeichert wird.

Ebenfalls als sehr vorteilhaft hat es sich erwiesen, wenn der Sammelbehälter mit der Auffangvorrichtung verbunden ist.

Hiermit bildet der Sammelbehälter zusammen mit der Auffangvorrichtung eine Einheit.

Eine weitere sehr vorteilhafte Ausgestaltung der Erfindung ist auch darin zu sehen, daß eine Schlauchverbindung zwischen Auffangvorrichtung und Sammelbehälter angeordnet ist.

Damit lässt sich der Sammelbehälter getrennt von der Auffangvorrichtung plazieren.

Äußerst vorteilhaft ist es, wenn der Sammelbehälter am Bein der Person befestigt ist.

Hierdurch stört der Sammelbehälter die tragende Person wenn überhaupt nur unwesentlich.

Dabei hat es sich als vorteilhaft erwiesen, wenn der Sammelbehälter mittels eines Gurtes am Körper befestigt ist.

Dadurch lässt sich die Befestigung besonders leicht anbringen und auf verschiedene Personen einstellen.

Eine weitere sehr vorteilhafte Fortbildung der Erfindung liegt auch darin, daß der Sammelbehälter öffenbar ausgebildet ist.

Es hat sich auch als sehr vorteilhaft erwiesen, wenn der Sammelbehälter eine Entleerungsmöglichkeit aufweist.

Hiermit lässt sich der Sammelbehälter wiederverwenden.

Besonders vorteilhaft ist es dabei auch, wenn ein verschließbares Ventil vorgesehen ist.

Damit lässt sich der Behälter besonders leicht entleeren.

Eine ebenfalls sehr vorteilhafte Weiterbildung der Erfindung liegt auch darin, daß der Sammelbehälter mit einem saugfähigen Material gefüllt ist.

Hierdurch wird auch bei einer eventuellen Beschädigung des Sammelbehälters ein versehentliches Auslaufen des gespeicherten Flüssigkeit wirkungsvoll vermieden.

Es hat sich dabei auch als sehr vorteilhaft erwiesen, wenn der Sammelbehälter eine Öffnung zur Entnahme des saugfähigen Materials aufweist.

Dadurch lässt sich der Behälter besonders leicht entleeren.

Sehr vorteilhaft ist es auch, wenn eine Einrichtung zum Desodorieren vorgesehen ist.

Hierdurch lassen sich nicht erwünschte Gerüche netralisieren bzw. überdecken.

Eine weitere, sehr vorteilhafte Fortbildung der Erfindung liegt auch darin, daß die Auffangvorrichtung aus einem wasserundurchlässigen Material gefertigt ist.

Es hat sich als sehr vorteilhaft erwiesen, wenn die Auffangvorrichtung in Form einer Hose ausgebildet ist.

Hiermit wird die austretende Flüssigkeit sicher aufgefangen.

Äußerst vorteilhaft ist es aber auch, wenn als Auffangvorrichtung ein in eine Hose eingebrachter Einsatz vorgesehen ist.

Damit kann zum Beispiel eine Unterhose die Funktion der Auffangvorrichtung mitübernehmen.

Eine besonders vorteilhafte Ausgestaltung der Erfindung liegt auch darin, daß der Einsatz aus einem flexiblen und wasserundurchlässigen Material gefertigt ist.

Hierdurch passt sich die Auffangvorrichtung besonder gut an die anatomischen Gegebenheiten des Körpers der tragenden Person an.

Im folgenden ist die Erfindung anhand eines Ausführungsbeispiels veranschaulicht. Dabei zeigt:
- Fig. 1: eine schematische Frontansicht einer Vorrichtung zum Auffangen und Sammeln von Urin und
- Fig. 2: eine schematische Seitenansicht derselben Vorrichtung.

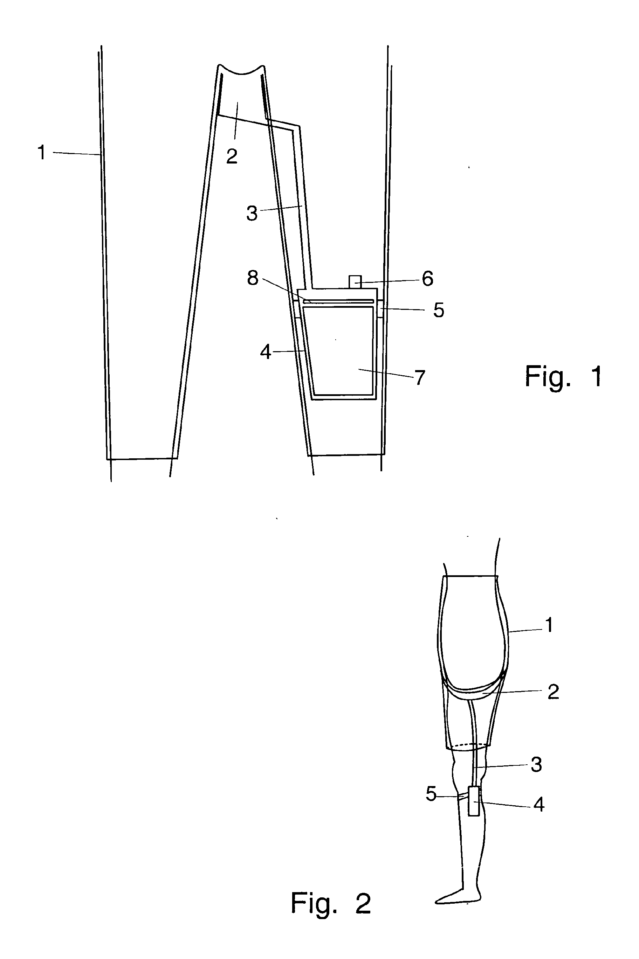

Mit 1 ist in Fig. 1 eine Unterhose mit einem wasserundurchlässigen Einsatz 2 im Bereich der Scheide der Frau bzw. des Penis des Mannes. Dieser Einsatz 2 nimmt austretenden Urin auf und leitet diesen mittels einer Schlauchverbindung 3 an einen Sammelbehälter 4 weiter, der den Urin aufnimmt.

Der Einsatz 2 kann aus einem steifen oder flexiblen Material gefertigt sein. Der Einsatz 2 kann in die Unterhose 1 eingenäht oder aber auch lösbar mit dieser verbunden sein.

Der Sammelbehälter 4 kann mit einem Gurt 5 am Bein der Person befestigt sein, Es ist aber auch denkbar, daß der Sammelbehälter 4 direkt an der Unterhose 1 befestigt ist, wie dies in Fig. 1 angedeutet ist. Die Befestigung des Sammelbehälters 4 am Gurt 5 bzw. an der Unterhose 1 kann lösbar ausgebildet sein.

Der Sammelbehälter 4 kann steif oder flexibel ausgebildet sein und kann eine Möglichkeit zur Öffnung aufweisen. Zusätzlich weist der Sammelbehälter 4 ein Ventil 6 zur Entleerung auf.

Es ist denkbar, daß der Sammelbehälter 4 mit einem saugfähigen Material 7 gefüllt ist, welches die aufgefangene Flüssigkeit aufsaugt und in seinem Inneren speichert. Sobald das Material 7 vollgesogen ist, kann es über eine Entnahmeöffnung 8 aus dem Sammelbehälter 4 entnommen werden.

Die Schlauchverbindung 3 zwischen dem Einsatz 2 und dem Sammelbehälter 4 kann sowohl am Einsatz 2 als auch am Sammelbehälter 4 lösbar befestigt sein.

Es ist auch denkbar, daß der Sammelbehälter 4 direkt am Einsatz 2 angeordnet ist und mit diesem eine Einheit bildet.

Desweiteren ist es auch denkbar, daß zwischen dem Einsatz 2 und dem Sammelbehälter 4 ein Ventil 9 vorgesehen ist, welches ein ungewolltes Rücklaufen von im Sammelbehälter gespeicherter Flüssigkeit zum Einsatz verhindert.

Ebenfalls denkbar ist es, daß am Sammelbehälter 4 ein nicht dargestelltes Ventil vorgesehen ist, welches beim Eintritt von Flüssigkeit in den Behälter verdrängte Luft nach außen entlässt.

## Patentansprüche

1. Vorrichtung zum Auffangen von Körperflüssigkeiten, insbesondere von Urin, insbesondere bei unter Inkontinenz leidenden Personen, **dadurch gekennzeichnet, daß** eine unter der Kleidung zu tragende Einrichtung(2) zum Auffangen des austretenden Urins vorgesehen ist.

2. Vorrichtung nach Anspruch 1, **dadurch gekennzeichnet, daß** ein Sammelbehälter(4) zum Speichern des aufgefangenen Urins vorgesehen ist.

3. Vorrichtung nach Anspruch 2, **dadurch gekennzeichnet, daß** der Sammelbehälter(4) mit der Auffangvorrichtung(2) verbunden ist.

4. Vorrichtung nach Anspruch 2 oder 3, **dadurch gekennzeichnet, daß** eine Schlauchverbindung(3) zwischen Auffangvorrichtung(2) und Sammelbehälter (4) angeordnet ist.

5. Vorrichtung nach Anspruch 2, 3 oder 4, **dadurch gekennzeichnet, daß** der Sammelbehälter(4) am Bein der Person befestigt ist.

6. Vorrichtung nach einem der Ansprüche 2 bis 5, **dadurch gekennzeichnet, daß** der Sammelbehälter(4) mittels eines Gurtes(5) am Körper befestigt ist.

7. Vorrichtung nach einem der Ansprüche 2 bis 6, **dadurch gekennzeichnet, daß** der Sammelbehälter(4) öffenbar ausgebildet ist.

8. Vorrichtung nach einem der Ansprüche 2 bis 7, **dadurch gekennzeichnet, daß** der Sammelbehälter(4) eine Entleerungsmöglichkeit aufweist.

9. Vorrichtung nach Anspruch 8, **dadurch gekennzeichnet, daß** ein verschließbares Ventil(6) vorgesehen ist.

10. Vorrichtung nach einem der Ansprüche 2 bis 9, **dadurch gekennzeichnet, daß** der Sammelbehälter(4) mit einem saugfähigen Material(7) gefüllt ist.

11. Vorrichtung nach einem der Ansprüche 2 bis 10, **dadurch gekennzeichnet, daß** der Sammelbehälter(4) eine Öffnung(8) zur Entnahme des saugfähigen Materials(7) aufweist.

12. Vorrichtung nach einem der vorangehenden Ansprüche, **dadurch gekennzeichnet, daß** eine Einrichtung zum Desodorieren vorgesehen ist.

13. Vorrichtung nach einem der vorangehenden Ansprüche, **dadurch gekennzeichnet, daß** die Auffangvorrichtung(2) aus einem wasserundurchlässigen Material gefertigt ist.

14. Vorrichtung nach einem der vorangehenden Ansprüche, **dadurch gekennzeichnet, daß** die Auffangvorrichtung in Form einer Hose ausgebildet ist.

15. Vorrichtung nach einem der vorangehenden Ansprüche, **dadurch gekennzeichnet, daß** als Auffangvorrichtung ein in eine Hose(1) eingebrachter Einsatz(2) vorgesehen ist.

16. Vorrichtung nach Anspruch 15, **dadurch gekennzeichnet, daß** der Einsatz(2) aus einem flexiblen und wasserundurchlässigen Material gefertigt ist.
